(19) Europäisches Patentamt
European Patent Office
Office européen des brevets

(11) Veröffentlichungsnummer: **0 530 616 A1**

(12) **EUROPÄISCHE PATENTANMELDUNG**

(21) Anmeldenummer: **92114288.1**

(51) Int. Cl.⁵: **C07D 403/12**, A01N 47/44

(22) Anmeldetag: **21.08.92**

(30) Priorität: **03.09.91 DE 4129317**

(43) Veröffentlichungstag der Anmeldung:
**10.03.93 Patentblatt 93/10**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Fest, Christa, Dr.**
**Im Johannistal 20**
**W-5600 Wuppertal(DE)**
Erfinder: **Santel, Hans-Joachim, Dr.**
**Grünstrasse 9a**
**W-5090 Leverkusen(DE)**
Erfinder: **Lürssen, Klaus, Dr.**
**August-Kierspel-Strasse 145**
**W-5060 Bergisch Gladbach 2(DE)**
Erfinder: **Schmidt, Robert R., Dr.**
**Im Waldwinkel 110**
**W-5060 Bergisch Gladbach 2(DE)**

(54) **Pyrazolylsulfonylguanidinopyrimidine als Herbizide.**

(57) Die Erfindung betrifft neue, substituierte Pyrazolylsulfonylguanidinopyrimidine der allgemeinen Formel (I)

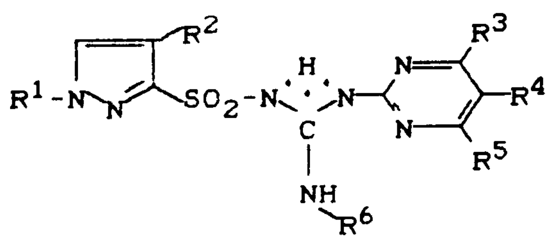

(I)

in welcher

| | |
|---|---|
| $R^1$ | für jeweils gegebenenfalls substituiertes Alkyl, Alkenyl oder Alkinyl steht, |
| $R^2$ | für Wasserstoff, Halogen oder jeweils gegebenenfalls substituiertes Alkyl oder Alkoxycarbonyl steht, |
| $R^3$, $R^4$ und $R^5$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, und |
| $R^6$ | für Amino oder die Gruppierungen -NH-A-$R^7$ oder |

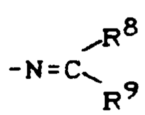

EP 0 530 616 A1

steht, worin

A für die Gruppierungen -CO-, -COO-, -CONH- oder $-SO_2-$ steht,

$R^7$ für jeweils gegebenenfalls substituiertes Alkyl, Aralkyl oder Aryl steht,

$R^8$ für Wasserstoff oder für jeweils gegebenenfalls substituiertes Alkyl, Aryl oder Aralkyl steht, und

$R^9$ für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^8$ für gegebenenfalls substituiertes Alkandiyl steht,

Verfahren zu ihrer Herstellung, neue Zwischenprodukte sowie ihre Verwendung als Herbizide.

Die Erfindung betrifft neue substituierte Pyrazolylsulfonylguanidinopyrimidine, Verfahren zu ihrer Herstellung und ihre Verwendung als Herbizide.

Es ist bereits bekannt, daß bestimmte Aminoguanidinoazine, wie z.B. N'-(4,6-Dimethyl-pyrimidin-2-yl)-N''-amino-N'''-(1-methyl-4-ethoxycarbonyl-pyrazol-5-yl-sulfonyl)-guanidin, herbizide Eigenschaften aufweisen (vgl. EP-A 343 462, vgl. auch EP-A 121 082, EP-A 302 378 und EP-A 414 067). Die herbizide Wirkung der bisher bekannten Aminoguanidinoazine ist jedoch nicht immer ganz zufriedenstellend.

Es wurden nun neue substituierte Pyrazolylsulfonylguanidinopyrimidine der allgemeinen Formel (I) gefunden,

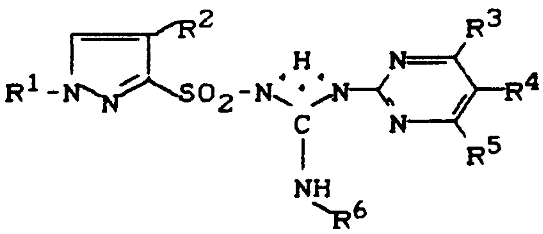

(I)

in welcher

| | |
|---|---|
| $R^1$ | für jeweils gegebenenfalls substituiertes Alkyl, Alkenyl oder Alkinyl steht, |
| $R^2$ | für Wasserstoff, Halogen oder jeweils gegebenenfalls substituiertes Alkyl oder Alkoxycarbonyl steht, |
| $R^3$, $R^4$ und $R^5$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, und |
| $R^6$ | für Amino oder die Gruppierungen -NH-A-$R^7$ oder 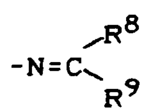 steht, worin |
| A | für die Gruppierungen -CO-, -COO-, -CONH- oder -$SO_2$- steht, |
| $R^7$ | für jeweils gegebenenfalls substituiertes Alkyl, Aralkyl oder Aryl steht, |
| $R^8$ | für Wasserstoff oder für jeweils gegebenenfalls substituiertes Alkyl, Aryl oder Aralkyl steht, und |
| $R^9$ | für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^8$ für gegebenenfalls substituiertes Alkandiyl steht. |

Die allgemeine Formel (I) steht für die einzelnen möglichen Tautomeren der Formeln (IA), (IB) und (IC) und für die Gemische dieser Tautomeren sowie für die E- und Z-Isomeren, welche durch die jeweils vorhandenen C-N-Doppelbindungen bedingt sind, und deren Gemische.

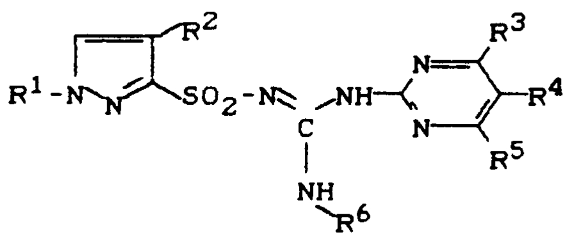

(IA)

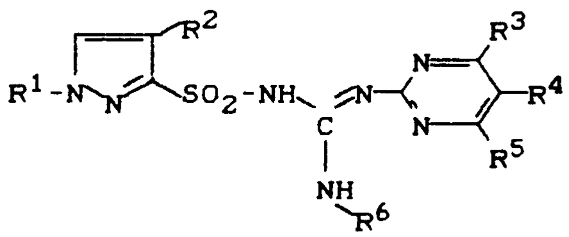

(IB)

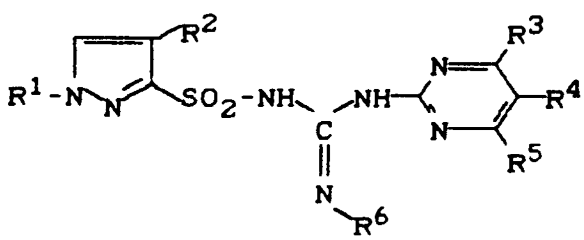

(IC)

Man erhält die neuen Pyrazolylsulfonylguanidinopyrimidine der allgemeinen Formel (I), wenn man

(a) Pyrazolylsulfonyl-iso(thio)ureidopyrimidine der allgemeinen Formel (II)

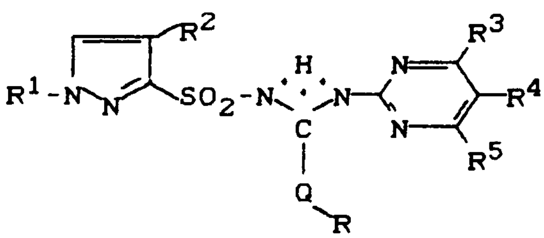

(II)

in welcher

| | |
|---|---|
| $R^1$, $R^2$, $R^3$, $R^4$ und $R^5$ | die oben angegebenen Bedeutungen haben, |
| Q | für Sauerstoff oder Schwefel steht und |
| R | für Alkyl steht, |

mit Aminoverbindungen der allgemeinen Formel (III)

$H_2N\text{-}R^6$ (III)

in welcher

$R^6$ die oben angegebene Bedeutung hat,

gegebenenfalls in Gegenwart eines Verdünnungsmittels umsetzt, oder wenn man

(b) für den Fall, daß in der Formel (I) $R^6$ für die Gruppierung $\text{-NH-A-}R^7$ steht und $R^1$, $R^2$, $R^3$, $R^4$, $R^5$, A und $R^7$ die oben angegebenen Bedeutungen haben,

Pyrazolylsulfonyl-aminoguanidinopyrimidine der allgemeinen Formel (Ia)

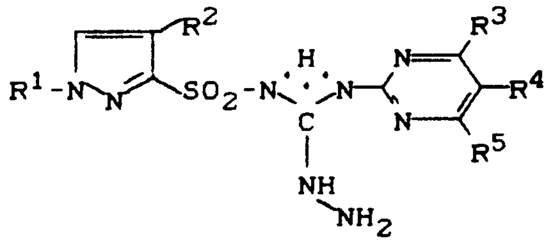 (Ia)

in welcher
$R^1$, $R^2$, $R^3$, $R^4$ und $R^5$ die oben angegebenen Bedeutungen haben,
mit Halogenverbindungen der allgemeinen Formel (IV)

$X-A-R^7$ (IV)

in welcher
A und $R^7$ die oben angegebenen Bedeutungen haben und
X für Halogen steht,
gegebenenfalls in Gegenwart eines Verdünnungsmittels und gegebenenfalls in Gegenwart eines Säureakzeptors umsetzt, oder wenn man
(c) für den Fall, daß in der Formel (I) $R^6$ für die Gruppierung

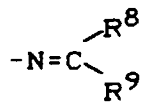

steht und $R^1$, $R^2$, $R^3$, $R^4$, $R^5$,
$R^8$ und $R^9$ die oben angegebenen Bedeutungen haben,
Pyrazolylsulfonyl-aminoguanidinopyrimidine der allgemeinen Formel (Ia)

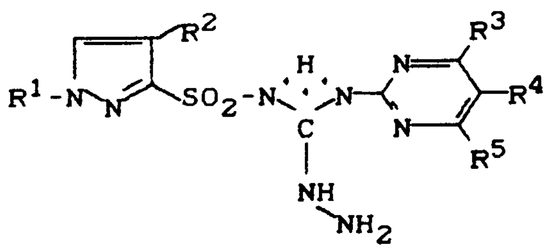 (Ia)

in welcher
$R^1$, $R^2$, $R^3$, $R^4$ und $R^5$ die oben angegebenen Bedeutungen haben,
mit Carbonylverbindungen der allgemeinen Formel (V)

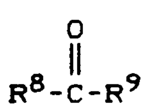 (V)

in welcher
$R^8$ und $R^9$ die oben angegebenen Bedeutungen haben,
oder mit N,N-Dialkyl-carbonsäureamid-acetalen bzw. -ketalen gegebenenfalls in Gegenwart eines Kondensationshilfsmittels und gegebenenfalls in Gegenwart eines Verdünnungsmittels umsetzt.

Die neuen substituierten Pyrazolylsulfonylguanidinopyrimidine zeichnen sich durch starke herbizide Wirksamkeit aus.

Überraschenderweise zeigen die neuen Verbindungen der allgemeinen Formel (I) erheblich stärkere herbizide Wirkung als vorbekannte Verbindungen ähnlicher Struktur und gleichartiger Wirkungsrichtung, wie z.B. die Verbindung N'-(4,6-Dimethyl-pyrimidin-2-yl)-N''-amino-N'''-(1-methyl-4-ethoxycarbonyl-pyrazol-5-yl-sulfonyl)-guanidin (vgl. EP-A 343 462).

Gegenstand der vorliegenden Erfindung sind vorzugsweise Verbindungen der Formel (I), in welcher

$R^1$ für jeweils gegebenenfalls durch Halogen, Cyano, $C_1$-$C_4$-Alkoxy oder $C_1$-$C_4$-Alkoxycarbonyl substituiertes $C_1$-$C_4$-Alkyl, $C_2$-$C_4$-Alkenyl oder $C_2$-$C_4$-Alkinyl steht,

$R^2$ für Wasserstoff, Halogen oder für jeweils gegebenenfalls durch Halogen oder $C_1$-$C_4$-Alkoxy substituiertes $C_1$-$C_4$-Alkyl oder $C_1$-$C_4$-Alkoxycarbonyl steht,

$R^3$, $R^4$ und $R^5$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino stehen, und

$R^6$ für Amino oder die Gruppierungen -NH-A-$R^7$ oder

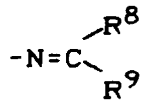

steht,

worin

A für die Gruppierungen -CO-, -COO-, -CONH- oder -$SO_2$- steht,

$R^7$ für gegebenenfalls durch Halogen substituiertes $C_1$-$C_{10}$-Alkyl oder für jeweils gegebenenfalls einfach bis dreifach substituiertes Benzyl, Phenylethyl, Phenyl oder Naphthyl steht, wobei die Substituenten vorzugsweise ausgewählt sind aus der Reihe Fluor, Chlor, Brom, Cyano, Nitro, Carboxy, Carbamoyl, jeweils gegebenenfalls durch Fluor, und/oder Chlor einfach bis dreifach substituiertes $C_1$-$C_4$-Alkyl, $C_1$-$C_4$-Alkoxy oder $C_1$-$C_4$-Alkylthio, jeweils gegebenenfalls durch Fluor, Chlor, Methoxy oder Ethoxy substituiertes $C_1$-$C_4$-Alkoxycarbonyl oder $C_1$-$C_4$-Alkylaminocarbonyl,

$R^8$ für Wasserstoff, für gegebenenfalls durch Fluor, Chlor und/oder Brom substituiertes $C_1$-$C_6$-Alkyl oder für jeweils gegebenenfalls durch Fluor, Chlor, Brom, $C_1$-$C_4$-Alkyl und/oder $C_1$-$C_4$-Alkoxy substituiertes Phenyl oder Benzyl steht, und

$R^9$ für Wasserstoff, für jeweils gegebenenfalls durch Fluor, Chlor und/oder Brom substituiertes $C_1$-$C_{20}$-Alkyl, $C_2$-$C_{10}$-Alkenyl, $C_2$-$C_{10}$-Alkinyl oder $C_4$-$C_{10}$-Alkadienyl, für $C_1$-$C_4$-Alkoxy-$C_1$-$C_4$-alkyl, für $C_1$-$C_4$-Alkylthio-$C_1$-$C_4$-alkyl, für gegebenenfalls durch Fluor, Chlor, Brom, Carboxy, Cyano, Nitro, Amino, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Di-($C_1$-$C_4$-alkyl)-amino, $C_1$-$C_4$-Alkoxycarbonyl, $C_1$-$C_4$-Alkoxy-$C_1$-$C_4$-alkoxycarbonyl und/oder Phenoxy (welches gegebenenfalls durch Fluor, Chlor und/oder Trifluormethyl substituiert ist) substituiertes Phenyl, für Naphthyl, für jeweils gegebenenfalls durch Cyano, Nitro, Fluor, Chlor, Brom, $C_1$-$C_4$-Alkyl und/oder $C_1$-$C_4$-Alkoxy substituiertes Pyridyl, Pyrrolyl, Furyl, Thiazolyl oder Thienyl, für Dithienyl, für jeweils gegebenenfalls durch Fluor, Chlor, Brom, $C_1$-$C_4$-Alkyl und/oder $C_1$-$C_4$-Alkoxy substituiertes Phenyl-$C_1$-$C_2$-alkyl oder Phenylethenyl, für $C_1$-$C_6$-Alkoxy, $C_1$-$C_6$-Alkoxycarbonyl oder Di-($C_1$-$C_4$-alkyl)-amino steht, oder zusammen mit $R^8$ für gegebenenfalls durch Fluor, Chlor, Brom, $C_1$-$C_4$-Alkyl und/oder $C_1$-$C_4$-Alkoxycarbonyl substituiertes $C_2$-$C_6$-Alkandiyl steht.

Die Erfindung betrifft insbesondere Verbindungen der Formel (I), in welcher

$R^1$ für Methyl, Ethyl, Propyl, Fluormethyl, Difluormethyl, Trifluormethyl, Chlormethyl, Dichlormethyl, Trichlormethyl, Fluorethyl, Difluorethyl, Trifluorethyl, Tetrafluorethyl, Pentafluorethyl, Chlorethyl, Dichlorethyl, Trichlorethyl, Chlorfluorethyl, Chlordifluorethyl, Chlortrifluorethyl, Cyanomethyl, Cyanoethyl, Methoxymethyl, Ethoxymethyl, Methoxyethyl, Ethoxyethyl, Methoxycarbonylmethyl, Ethoxycarbonylmethyl, Methoxycarbonylethyl, Ethoxycarbonylethyl, Allyl, Chlorallyl, Dichlorallyl, Trichlorallyl oder Propargyl steht,

$R^2$ für Wasserstoff, Chlor, Brom, Methyl, Methoxycarbonyl oder Ethoxycarbonyl steht,

$R^3$ für Wasserstoff, Fluor, Chlor, Brom, Methyl, Ethyl, Trifluormethyl, Chlormethyl, Methoxymethyl, Methoxy, Ethoxy, Difluormethoxy, Trifluorethoxy, Methoxyethoxy, Methylthio, Methylamino, Ethylamino oder Dimethylamino steht,

$R^4$ für Wasserstoff, Fluor, Chlor, Brom, Methyl, Trifluormethyl oder Methoxy steht,

$R^5$ für Wasserstoff, Fluor, Chlor, Brom, Methyl, Ethyl, Trifluormethyl, Methoxy, Ethoxy oder Difluormethoxy steht, und

$R^6$ für Amino oder die Gruppierungen
-NH-A-$R^7$ oder

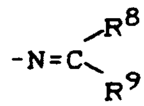

steht, worin

A für die Gruppierungen -CO-, -COO-, -CONH- oder -$SO_2$- steht,

$R^7$ für gegebenenfalls durch Fluor oder Chlor substituiertes $C_1$-$C_6$-Alkyl oder für jeweils gegebenenfalls einfach oder zweifach substituiertes Benzyl oder Phenyl steht, wobei die Substituenten vorzugsweise ausgewählt sind aus der Reihe Fluor, Chlor, Brom, Carboxy, Methyl, Trifluormethyl, Methoxy, Difluormethoxy, Trifluormethoxy, Methoxycarbonyl, Ethoxycarbonyl,

$R^8$ für Wasserstoff, $C_1$-$C_4$-Alkyl oder Phenyl steht,

$R^9$ für $C_1$-$C_{10}$-Alkyl, $C_2$-$C_{10}$-Alkenyl oder $C_4$-$C_{10}$-Alkadienyl, für gegebenenfalls durch Fluor, Chlor, Brom, Carboxy, Cyano, Nitro, Methyl, Ethyl, Trifluormethyl, Methoxy, Ethoxy, Difluormethoxy, Trifluormethoxy, Methylthio, Ethylthio, Difluormethylthio, Trifluormethylthio, $C_1$-$C_4$-Alkoxycarbonyl, Dimethylamino, $C_1$-$C_2$-Alxoxy-$C_1$-$C_2$-alkoxycarbonyl und/oder Phenoxy (welches gegebenenfalls durch Fluor, Chlor und/oder Trifluormethyl substituiert ist) substituiertes Phenyl, für jeweils gegebenenfalls durch Cyano, Nitro, Fluor, Chlor, Brom, Methyl, Ethyl, Methoxy oder Ethoxy substituiertes Pyridyl, Furyl, Thiazolyl oder Thienyl, für Dithienyl, für Benzyl oder Phenylethenyl, für $C_1$-$C_4$-Alkoxy, $C_1$-$C_4$-Alkoxycarbonyl oder Dimethylamino, oder zusammen mit $R^8$ für Butan-1,4-diyl (Tetramethylen) oder Pentan-1,5-diyl (Pentamethylen)

steht.

Verwendet man beispielsweise N'-(4-Methoxy-6-methyl-pyrimidin-2-yl)-N''-(4-chlor-1-methyl-pyrazol-3-yl-sulfonyl)-O-methyl-isoharnstoff und Hydrazin als Ausgangsstoffe, so kann der Reaktionsablauf beim erfindungsgemäßen Verfahren (a) durch das folgende Formelschema skizziert werden:

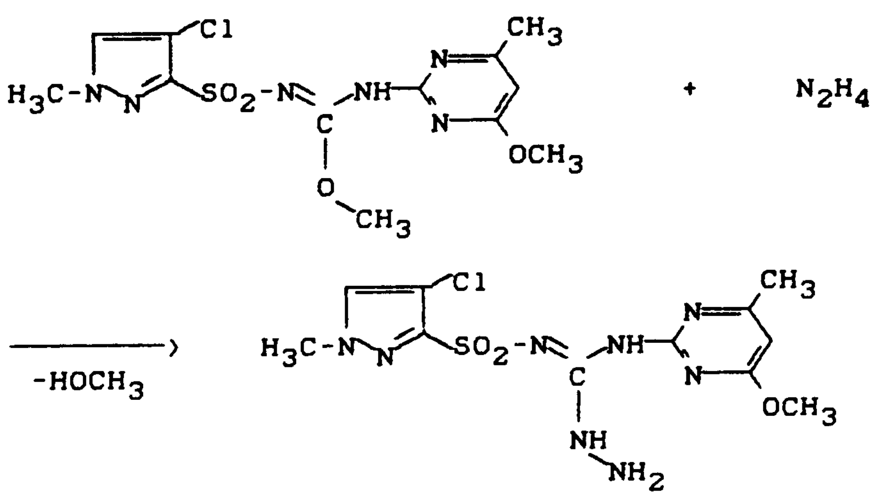


Verwendet man beispielsweise N'-(4,6-Dimethoxy-pyrimidin-2-yl)-N''-amino-N'''-(4-methoxycarbonyl-1-trifluormethyl-pyrazol-3-yl-sulfonyl)-guanidin und Methansulfonsäurechlorid als Ausgangsstoffe, so kann der Reaktionsablauf beim erfindungsgemäßen Verfahren (b) durch das folgende Formelschema skizziert wer-

den:

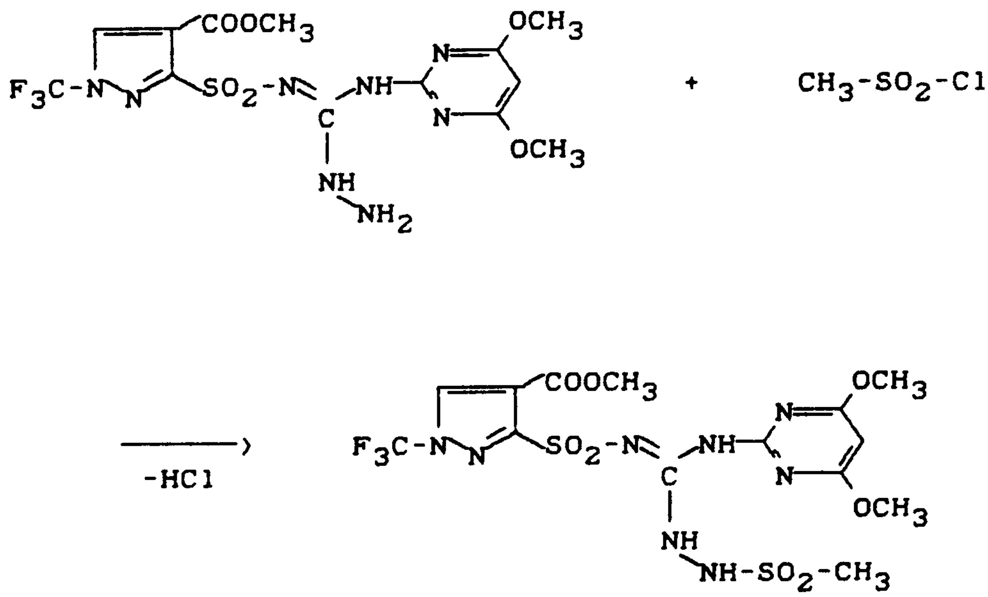


Verwendet man beispielsweise N'-(4-Methyl-pyrimidin-2-yl)-N''-amino-N'''-(4-brom-1-ethyl-pyrazol-3-yl-sulfonyl)-guanidin und Benzaldehyd als Ausgangsstoffe, so kann der Reaktionsablauf beim erfindungsgemäßen Verfahren (c) durch das folgende Formelschema skizziert werden:

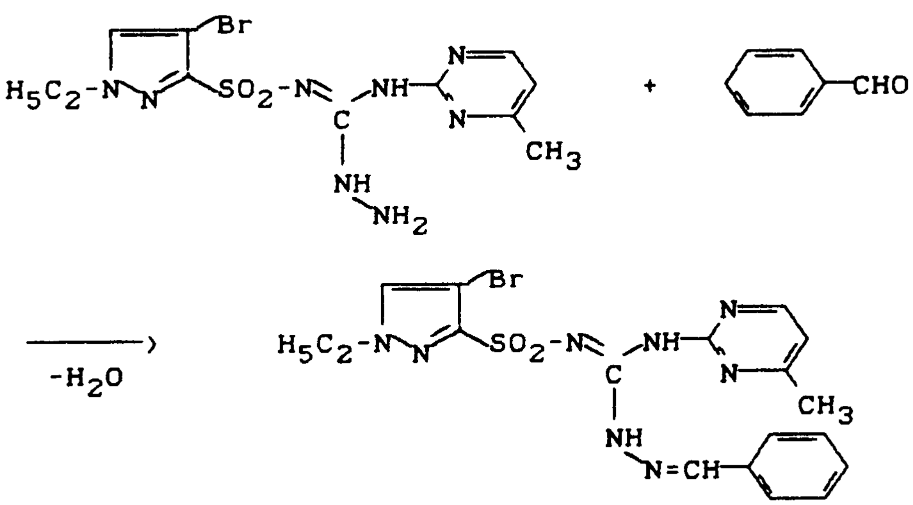


Die beim erfindungsgemäßen Verfahren (a) zur Herstellung von Verbindungen der Formel (I) als Ausgangsstoffe zu verwendenden Pyrazolylsulfonyl-iso(thio)ureido-pyrimidine sind durch die Formel (II) allgemein definiert.

In Formel (II) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden; weiter stehen in Formel (II) vorzugsweise

Q für Sauerstoff oder Schwefel und

R für Methyl.

Die Ausgangsstoffe der Formel (II) sind noch nicht aus der Literatur bekannt und sind ebenfalls Gegenstand der vorliegenden Patentanmeldung.

Man erhält die neuen Pyrazolylsulfonyl-iso(thio)-ureido-pyrimidine der Formel (II), wenn man Pyrazolsulfonsäurechloride der allgemeinen Formel (VI)

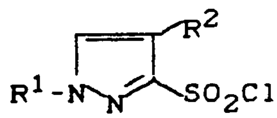

(VI)

in welcher

$R^1$ und $R^2$ die oben angegebenen Bedeutungen haben,

mit Iso(thio)harnstoffen der allgemeinen Formel (VII)

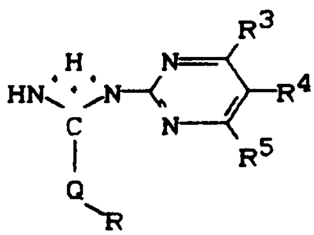

(VII)

in welcher

Q, R, $R^3$, $R^4$ und $R^5$ die oben angegebene Bedeutung haben,

gegebenenfalls in Gegenwart eines Säureakzeptors, wie z.B. 1,4-Diazabicyclo-[2,2,2]-octan (DABCO), und gegebenenfalls in Gegenwart eines Verdünnungsmittels, wie z.B. Methylenchlorid, bei Temperaturen zwischen -20 °C und +50 °C umsetzt (vgl. Herstellungsbeispiele).

In Formel (VI) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Pyrazolsulfonsäurechloride der Formel (VI) sind bekannt und/oder können nach an sich bekannten Verfahren hergestellt werden (vgl. J. Heterocycl. Chem. 21 (1984), 1017-1021).

In Formel (VII) haben $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^3$, $R^4$ und $R^5$ angegeben wurden;

weiter stehen in Formel (VII) vorzugsweise

Q für Sauerstoff oder Schwefel und

R für Methyl.

Die Iso(thio)harnstoffe der Formel (VII) sind bekannt und/oder können nach an sich bekannten Verfahren hergestellt werden (vgl. EP-A 358018, EP-A 416319).

Die beim erfindungsgemäßen Verfahren (a) zur Herstellung von Verbindungen der Formel (I) weiter als Ausgangsstoffe zu verwendenden Aminoverbindungen sind durch die Formel (III) allgemein definiert.

In Formel (III) hat $R^6$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^6$ angegeben wurde.

Die Ausgangsstoffe der Formel (III) sind bekannte Synthesechemikalien.

Die bei den erfindungsgemäßen Verfahren (b) und (c) zur Herstellung von Verbindungen der Formel (I) als Ausgangsstoffe zu verwendenden Pyrazolylsulfonyl-aminoguanidinopyrimidine sind durch die Formel (Ia) allgemein definiert.

In Formel (Ia) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden.

Die Pyrazolylsulfonyl-aminoguanidinopyrimidine der Formel (Ia) sind eine Teilmenge der erfindungsgemäßen neuen Verbindungen der Formel (I); sie können nach dem erfindungsgemäßen Verfahren (a), wenn $-R^6$ für $NH_2$- steht, hergestellt werden.

Die beim erfindungsgemäßen Verfahren (b) weiter als Ausgangsstoffe zu verwendenden Halogenverbindungen sind durch die Formel (IV) allgemein definiert.

In Formel (IV) haben A und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A und $R^7$ angegeben wurden;

X steht vorzugsweise für Chlor.

Die Ausgangsstoffe der Formel (IV) sind bekannte Synthesechemikalien.

Die beim erfindungsgemäßen Verfahren (c) weiter als Ausgangsstoffe zu verwendenden Carbonylverbindungen sind durch die Formel (V) allgemein definiert.

In Formel (V) haben $R^8$ und $R^9$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^8$ und $R^9$ angegeben wurden.

N,N-Dialkylcarbonsäureamid-acetale bzw. N,N-Dialkylcarbonsäureamid-ketale, welche weiter als Ausgangsstoffe beim erfindungsgemäßen Verfahren (c) eingesetzt werden können, sind vorzugsweise N,N-Dialkyl-formamid- oder -acetamid-acetale bzw. -ketale mit an N- bzw. O-gebundenen geradkettigen oder verzweigten Alkylresten mit 1 bis 4 Kohlenstoffatomen, wie z.B. N,N-Dimethyl- und N,N-Diethyl-formamid- und -acetamiddimethylacetal, -diethylacetal, -dipropylacetal, -diisopropylacetal, -dibutylacetal, -diisobutylacetal, -di-sec-butyl-acetal und -di-tert-butyl-acetal.

Die Ausgangsstoffe der Formel (V) sind bekannte Synthesechemikalien.

Das erfindungsgemäße Verfahren (a) zur Herstellung der neuen Pyrazolylsulfonylguanidinopyrimidine der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butanol, tert-Butanol, Glycoldimethylether, Diglycoldimethylether, Tetrahydrofuran, Dioxan, Essigsäuremethylester, Essigsäureethylester, Acetonitril, Propionitril, Dimethylformamid, Dimethylacetamid, N-Methylpyrrolidon, Dimethylsulfoxid und Tetramethylensulfon, in Betracht.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calciumhydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen -50 °C und +100 °C, vorzugsweise bei Temperaturen zwischen -20 °C und +50 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in größerem Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Das erfindungsgemäße Verfahren (c) zur Herstellung der neuen Verbindungen der Formel (I) wird gegebenenfalls unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Alkohole wie Methanol, Ethanol, Propanol, Isopropanol, Butanol und Isobutanol, Ester wie Essigsäuremethylester und -ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (c) wird gegebenenfalls in Gegenwart eines Kondensationshilfsmittels durchgeführt. Als solche kommen vorzugsweise die in der organischen Chemie üblichen Trockenmittel in Betracht. Hierzu gehören vorzugsweise wasserfreie Salze, wie z.B. Natriumsulfat, Magnesiumsulfat und Kaliumcarbonat.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 80 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in größerem Überschuß zu verwenden. Die Reaktionen werden im allgemeinen in einem geeigneten Verdünnungsmittel, gegebenenfalls in Gegenwart eines Kondensationshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich sehr gut zur Bekämpfung von monokotylen und dikotylen Unkräutern sowohl im Vorauflauf- als auch im Nachauflauf-Verfahren.

In gewissem Umfang zeigen sie auch fungizide Wirkung, insbesondere gegen Pyricularia oryzae am Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

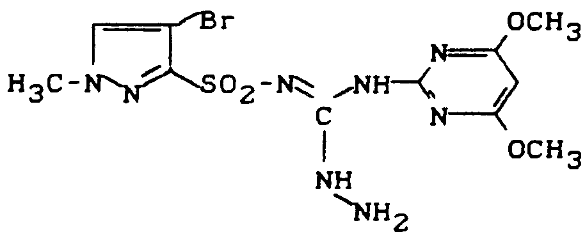

(Verfahren (a))

25,2 g (60 mMol) N-(4,6-Dimethoxy-pyrimidin-2-yl)-N'-(4-brom-1-methyl-pyrazol-3-yl-sulfonyl)-S-methylisothioharnstoff werden mit 150 ml Ethanol verrührt und dann mit 3,3 ml (66 mMol) Hydrazinhydrat versetzt. Das Reaktionsgemisch wird 20 Stunden bei 20 °C gerührt. Das dabei kristallin anfallende Produkt wird schließlich durch Filtrieren isoliert.

Man erhält 21,8 g (83,5% der Theorie) N'-(4,6-Dimethoxy-pyrimidin-2-yl)-N''-amino-N'''-(4-brom-1-methyl-pyrazol-3-yl-sulfonyl)-guanidin vom Schmelzpunkt 194 °C.

Beispiel 2

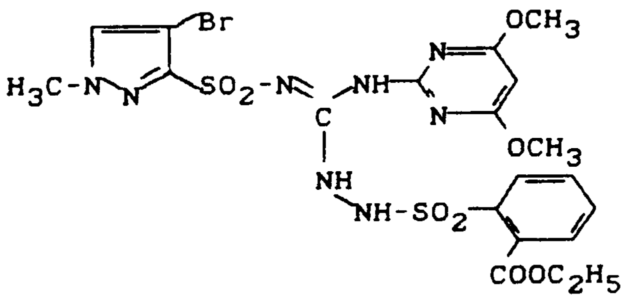

(Verfahren (b))

6,5 g (15 mMol) N'-(4,6-Dimethoxy-pyrimidin-2-yl)-N''-amino-N'''-(4-brom-1-methyl-pyrazol-3-yl-sulfonyl)-guanidin werden in 100 ml Methylenchlorid gelöst und nach Abkühlen auf -5 ° C mit 3,7 g (15 mMol) 2-Ethoxycarbonyl-benzolsulfonsäurechloridund dann mit 1,7 g 1,4-Diazabicyclo-[2,2,2]-octan (DABCO) versetzt. Das Reaktionsgemisch wird eine Stunde bei -5 ° C und dann 15 Stunden bei 20 ° C gerührt, dann 2mal mit je 100 ml Wasser gewaschen, mit Natriumsulfat getrocknet und filtriert. Das Filtrat wird im Wasserstrahlvakuum eingeengt und der feste Rückstand aus Acetonitril umkristallisiert.

Man erhält 1,2 g (12% der Theorie) N'-(4,6-Dimethoxy-pyrimidin-2-yl)-N''-(2-ethoxycarbonyl-phenylsulfonylamino)-N'''-(4-brom-1-methyl-pyrazol-3-yl-sulfonyl)-guanidin vom Schmelzpunkt 189 ° C.

Beispiel 3

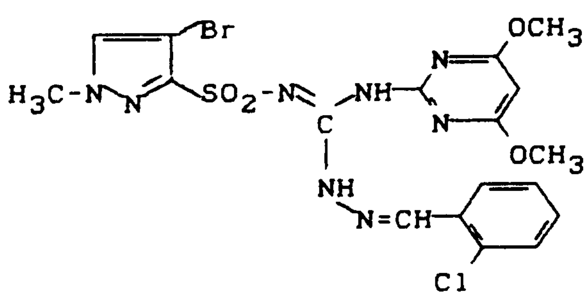

(Verfahren (c))

5,2 g (12 mMol) N'-(4,6-Dimethoxy-pyrimidin-2-yl)-N''-amino-N'''-(4-brom-1-methyl-pyrazol-3-yl-sulfonyl)-guanidin werden in 75 ml Ethanol aufgenommen und mit 1,7 g (12 mMol) 2-Chlor-benzaldehyd versetzt. Das Reaktionsgemisch wird 3 Stunden unter Rückfluß erhitzt und schließlich das kristallin anfallende Produkt durch Filtrieren isoliert.

Man erhält 6 g (89,5% der Theorie) N'-(4,6-Dimethoxy-pyrimidin-2-yl)-N''-(2-chlor-benzylidenamino)-N'''-(4-brom-1-methyl-pyrazol-3-yl-sulfonyl)-guanidin vom Schmelzpunkt 253 ° C.

Analog zu den Herstellungsbeispielen 1, 2 und 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

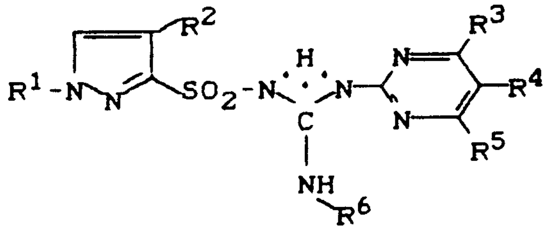

(I)

Tabelle 1: Beispiele für die Verbindungen der Formel (I)

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 4 | $CH_3$ | $COOC_2H_5$ | $OCH_3$ | H | $OCH_3$ | $NH_2$ | 169 |
| 5 | $CH_3$ | $COOC_2H_5$ | $OCH_3$ | H | $OCH_3$ | $-NH-SO_2-$ 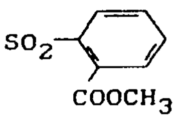 | 220 |
| 6 | $CH_3$ | Br | $OCH_3$ | H | $OCH_3$ | $-NH-SO_2-$ 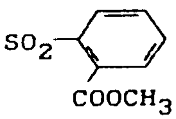 | 192 |
| 7 | $CH_3$ | Br | $OCH_3$ | H | $OCH_3$ | $-NH-SO_2-$ 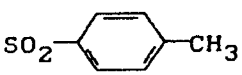 | 199 |
| 8 | $CH_3$ | Br | $CH_3$ | H | $OCH_3$ | $NH_2$ | 196 |
| 9 | $CH_3$ | $COOC_2H_5$ | $OCH_3$ | H | $OCH_3$ | $-NH-SO_2-$ 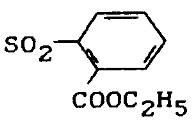 | 212 |
| 10 | $CH_3$ | $COOC_2H_5$ | $OCH_3$ | H | $OCH_3$ | $-NH-SO_2-$ 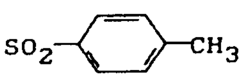 | 195 |
| 11 | $CH_3$ | $COOC_2H_5$ | $OCH_3$ | H | $OCH_3$ | $-NH-SO_2-CH_3$ | 221 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 12 | $CH_3$ | $COOC_2H_5$ | $CH_3$ | H | $OCH_3$ | $NH_2$ | 181 |
| 13 | $CH_3$ | Br | $CH_3$ | H | $OCH_3$ | $-NH-SO_2-$ 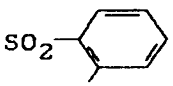 $COOCH_3$ | 195 |
| 14 | $CH_3$ | Br | $CH_3$ | H | $OCH_3$ | $-NH-SO_2-$ 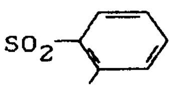 $COOC_2H_5$ | 177 |
| 15 | $CH_3$ | Br | $OCH_3$ | H | $OCH_3$ | $-NH-SO_2-$ 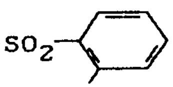 $COOCH(CH_3)_2$ | 165 |
| 16 | $CH_3$ | Br | $OCH_3$ | H | $OCH_3$ | $-NH-SO_2-$ 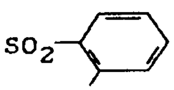 $COOC_3H_7$ | 167 |
| 17 | $CH_3$ | Br | $OCH_3$ | H | $OCH_3$ | $-NH-SO_2-$ 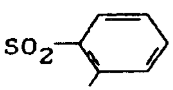 $COOCH(CH_3)C_2H_5$ | 110 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 18 | $CH_3$ | Br | $CH_3$ | H | $OCH_3$ | 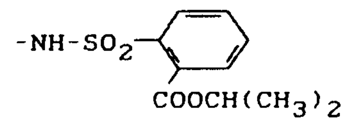 | 168 |
| 19 | $CH_3$ | Br | $CH_3$ | H | $OCH_3$ | 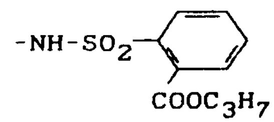 | 160 |
| 20 | $CH_3$ | Br | $CH_3$ | H | $OCH_3$ | 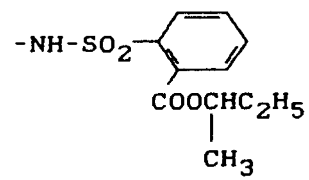 | 182 |
| 21 | $CH_3$ | Br | $CH_3$ | H | $OCH_3$ | 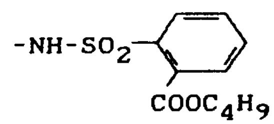 | 126 |
| 22 | $CH_3$ | Br | $OCH_3$ | H | $OCH_3$ | 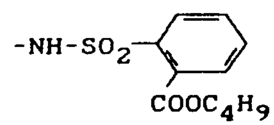 | 131 |

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 23 | $CH_3$ | $COOC_2H_5$ | $OCH_3$ | H | $OCH_3$ | 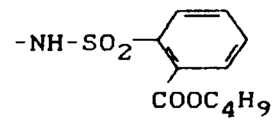 | 185 |
| 24 | $CH_3$ | $COOC_2H_5$ | $OCH_3$ | H | $OCH_3$ | 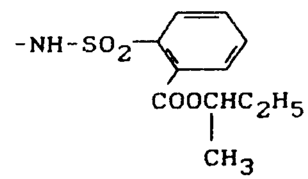 | 204 |
| 25 | $CH_3$ | $COOC_2H_5$ | $CH_3$ | H | $OCH_3$ | 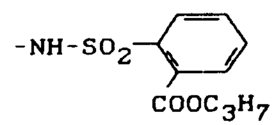 | 211 |
| 26 | $CH_3$ | $COOC_2H_5$ | $CH_3$ | H | $OCH_3$ | 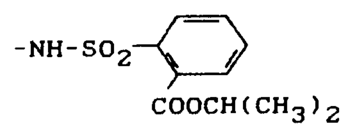 | 210 |
| 27 | $CH_3$ | $COOC_2H_5$ | $CH_3$ | H | $OCH_3$ | 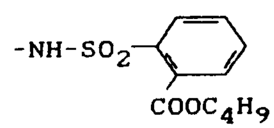 | 164 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 28 | $CH_3$ | $COOC_2H_5$ | $CH_3$ | H | $OCH_3$ | $-NH-SO_2-$ 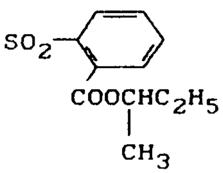 | 192 |
| 29 | $CH_3$ | $COOC_2H_5$ | $CH_3$ | H | $OCH_3$ | $-NH-SO_2-$ 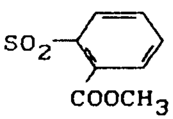 | 228 |
| 30 | $CH_3$ | $COOC_2H_5$ | $CH_3$ | H | $OCH_3$ | $-NH-SO_2-$ 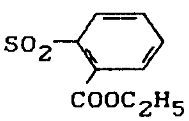 | 230 |
| 31 | $CH_3$ | $COOC_2H_5$ | $CH_3$ | H | $OCH_3$ | $-NH-SO_2-CH_3$ | 223 |
| 32 | $CH_3$ | Br | $CH_3$ | H | $OCH_3$ | $-NH-SO_2-$ 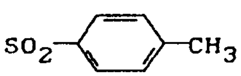 | 116 |
| 33 | $CH_3$ | Br | $CH_3$ | H | $OCH_3$ | $-NH-SO_2-CH_3$ | 185 |
| 34 | $CH_3$ | $COOC_2H_5$ | $OCH_3$ | H | $OCH_3$ | $-NH-SO_2-$ 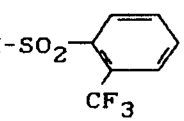 | 202 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 35 | $CH_3$ | $COOC_2H_5$ | $OCH_3$ | H | $OCH_3$ | $-NH-SO_2-$ 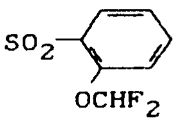 | 190 |
| 36 | $CH_3$ | $COOC_2H_5$ | $OCH_3$ | H | $OCH_3$ | $-NH-SO_2-C_2H_5$ | 223 |
| 37 | $CH_3$ | $COOC_2H_5$ | $OCH_3$ | H | $OCH_3$ | $-NH-SO_2-CH_2-$ 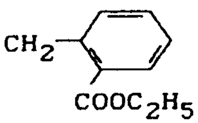 | 183 |
| 38 | $CH_3$ | $COOC_2H_5$ | $OCH_3$ | H | $OCH_3$ | $-NH-SO_2-CH_2-$ 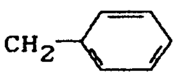 | 153 |
| 39 | $CH_3$ | $COOC_2H_5$ | $OCH_3$ | H | $OCH_3$ | $-NH-SO_2-$ 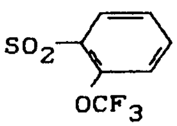 | 121 |
| 40 | $CH_3$ | $COOC_2H_5$ | $OCH_3$ | H | $OCH_3$ | $-NH-SO_2-$ 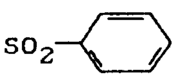 | 197 |
| 41 | $CH_3$ | $COOC_2H_5$ | $OCH_3$ | H | $OCH_3$ | $-NH-SO_2-$ 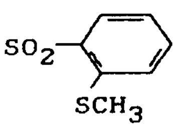 | 207 |

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 42 | $CH_3$ | $COOC_2H_5$ | $OCH_3$ | H | $OCH_3$ | $-NH-SO_2-CH(CH_3)_2$ | 171 |
| 43 | $CH_3$ | $COOC_2H_5$ | $OCH_3$ | H | $OCH_3$ | $-NH-SO_2-C_3H_7$ | 177 |
| 44 | $CH_3$ | $COOC_2H_5$ | $OCH_3$ | H | $OCH_3$ | $-NH-SO_2-CH_2-$ 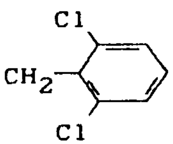 | 133 |
| 45 | $CH_3$ | $COOC_2H_5$ | $OCH_3$ | H | $OCH_3$ | $-NH-SO_2-$ 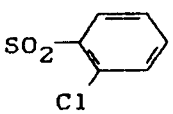 | 204 |
| 46 | $CH_3$ | $COOC_2H_5$ | $OCH_3$ | H | $OCH_3$ | $-NH-SO_2-(CH_2)_4Cl$ | 103 |
| 47 | $CH_3$ | $COOC_2H_5$ | $OCH_3$ | H | $OCH_3$ | $-NH-SO_2-N(CH_3)_2$ | 181 |
| 48 | $CH_3$ | $COOC_2H_5$ | $OCH_3$ | H | $OCH_3$ | $-NH-SO_2-C_4H_9$ | 196 |
| 49 | $CH_3$ | Br | $CH_3$ | H | $OCH_3$ | $-NH-SO_2-$ 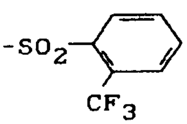 | 118 |
| 50 | $CH_3$ | Br | $CH_3$ | H | $OCH_3$ | $-NH-SO_2-$ 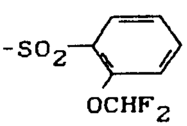 | 97 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 51 | $CH_3$ | Br | $CH_3$ | H | $OCH_3$ | $-NH-SO_2-C_2H_5$ | 103 |
| 52 | $CH_3$ | Br | $OCH_3$ | H | $OCH_3$ | 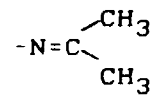 | 234 |
| 53 | $CH_3$ | Br | $CH_3$ | H | $OCH_3$ | 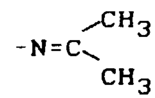 | 260 |
| 54 | $CH_3$ | Br | $CH_3$ | H | $OCH_3$ | 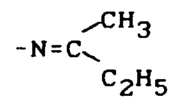 | 242 |
| 55 | $CH_3$ | Br | $CH_3$ | H | $OCH_3$ | 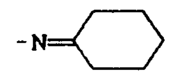 | 269 |
| 56 | $CH_3$ | $COOC_2H_5$ | $CH_3$ | H | $CH_3$ | 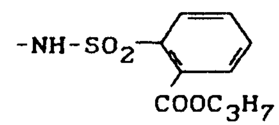 | 221 |
| 57 | $CH_3$ | $COOC_2H_5$ | $CH_3$ | H | $CH_3$ | 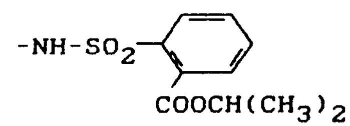 | 245 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 58 | $CH_3$ | $COOC_2H_5$ | $CH_3$ | H | $CH_3$ | $-NH-SO_2-$ 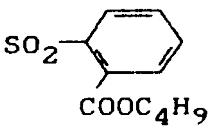 | 226 |
| 59 | $CH_3$ | $COOC_2H_5$ | $CH_3$ | H | $CH_3$ | $-NH-SO_2-$ 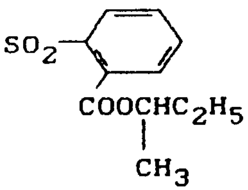 | 258 |
| 60 | $CH_3$ | Br | $CH_3$ | H | $OCH_3$ | $-NH-SO_2-CH_2-$ 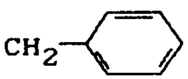 | 192 |
| 61 | $CH_3$ | Br | $CH_3$ | H | $OCH_3$ | $-NH-SO_2-CH_2-$ 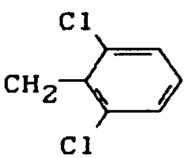 | 193 |
| 62 | $CH_3$ | Br | $CH_3$ | H | $OCH_3$ | $-NH-SO_2-C_3H_7$ | 147 |
| 63 | $CH_3$ | Br | $CH_3$ | H | $OCH_3$ | $-NH-SO_2-$ 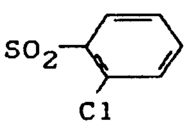 | 191 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 64 | $CH_3$ | $COOC_2H_5$ | $CH_3$ | H | $OCH_3$ | $-NH-SO_2-$ 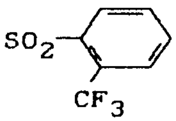 | 199 |
| 65 | $CH_3$ | $COOC_2H_5$ | $CH_3$ | H | $OCH_3$ | $-NH-SO_2-$ 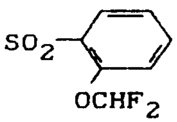 | 170 |
| 66 | $CH_3$ | $COOC_2H_5$ | $CH_3$ | H | $OCH_3$ | $-NH-SO_2-C_2H_5$ | 206 |
| 67 | $CH_3$ | $COOC_2H_5$ | $CH_3$ | H | $OCH_3$ | $-NH-SO_2-CH_2-$ 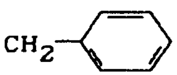 | 155 |
| 68 | $CH_3$ | $COOC_2H_5$ | $CH_3$ | H | $OCH_3$ | $-NH-SO_2-$ 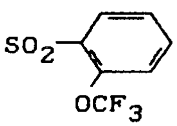 | 201 |
| 69 | $CH_3$ | $COOC_2H_5$ | $CH_3$ | H | $OCH_3$ | $-NH-SO_2-$ 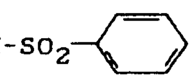 | 202 |
| 70 | $CH_3$ | $COOC_2H_5$ | $CH_3$ | H | $OCH_3$ | $-NH-SO_2-$ 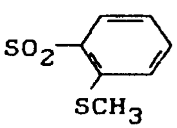 | 214 |

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 71 | $CH_3$ | $COOC_2H_5$ | $CH_3$ | H | $OCH_3$ | $-NH-SO_2-C_3H_7$ | 166 |
| 72 | $CH_3$ | $COOC_2H_5$ | $CH_3$ | H | $OCH_3$ | $-NH-SO_2$ 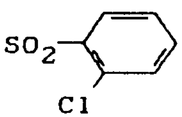 | 213 |
| 73 | $CH_3$ | $COOC_2H_5$ | $CH_3$ | H | $OCH_3$ | $-NH-SO_2-C_4H_9$ | 71 |
| 74 | $CH_3$ | $COOC_2H_5$ | $CH_3$ | H | $OCH_3$ | $-NH-SO_2$ 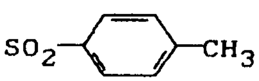 | 195 |
| 75 | $CH_3$ | $COOC_2H_5$ | $CH_3$ | H | $CH_3$ | $NH_2$ | 204 |
| 76 | $CH_3$ | $COOC_2H_5$ | $CH_3$ | H | $CH_3$ | $-NH-SO_2$ 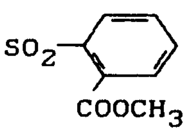 | 211 |
| 77 | $CH_3$ | $COOC_2H_5$ | $CH_3$ | H | $CH_3$ | $-NH-SO_2$ 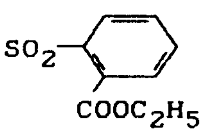 | 213 |
| 78 | $CH_3$ | $COOC_2H_5$ | $CH_3$ | H | $CH_3$ | $-NH-SO_2$ 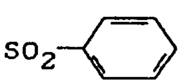 | 193 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 79 | $CH_3$ | $COOC_2H_5$ | $CH_3$ | H | $CH_3$ | $-NH-SO_2-CH_3$ | 206 |
| 80 | $CH_3$ | $COOC_2H_5$ | $CH_3$ | H | $CH_3$ | 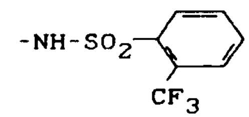 | 193 |
| 81 | $CH_3$ | $COOC_2H_5$ | $CH_3$ | H | $CH_3$ | 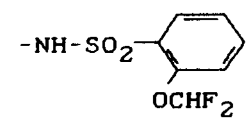 | 174 |
| 82 | $CH_3$ | $COOC_2H_5$ | $CH_3$ | H | $CH_3$ | 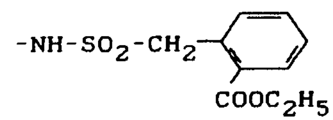 | 190 |
| 83 | $CH_3$ | $COOC_2H_5$ | $CH_3$ | H | $CH_3$ | 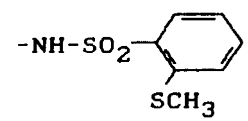 | 230 |
| 84 | $CH_3$ | Br | $CH_3$ | H | $CH_3$ | 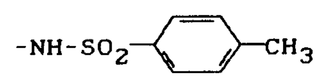 | 230 |
| 85 | $CH_3$ | Br | $CH_3$ | H | $OCH_3$ | 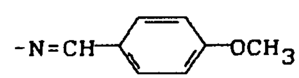 | 282 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 86 | $CH_3$ | Br | $CH_3$ | H | $OCH_3$ | -N=CH- 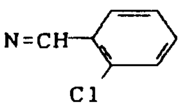 | 246 |
| 87 | $CH_3$ | Br | $CH_3$ | H | $OCH_3$ | -N=CH- 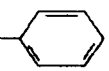 | 236 |
| 88 | $CH_3$ | Br | $OCH_3$ | H | $OCH_3$ | -N=CH- 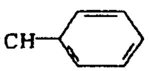 | 245 |
| 89 | $CH_3$ | Br | $OCH_3$ | H | $OCH_3$ | -N=CH- 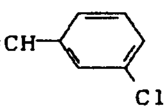 | 242 |
| 90 | $CH_3$ | Br | $OCH_3$ | H | $OCH_3$ | -N=CH- 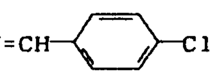 | 262 |
| 91 | $CH_3$ | Br | $OCH_3$ | H | $OCH_3$ | -N=CH- 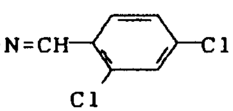 | 289 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 92 | $CH_3$ | Br | $OCH_3$ | H | $OCH_3$ | 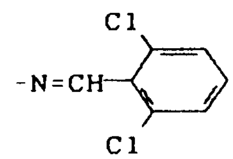 | 251 |
| 93 | $CH_3$ | Br | $OCH_3$ | H | $OCH_3$ | 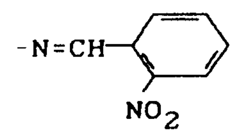 | 237 |
| 94 | $CH_3$ | Br | $OCH_3$ | H | $OCH_3$ | 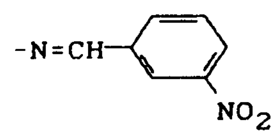 | 253 |
| 95 | $CH_3$ | Br | $OCH_3$ | H | $OCH_3$ | 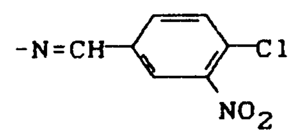 | 256 |
| 96 | $CH_3$ | Br | $OCH_3$ | H | $OCH_3$ | 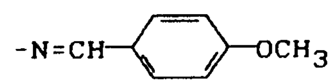 | 280 |
| 97 | $CH_3$ | Br | $CH_3$ | H | $CH_3$ | $NH_2$ | 189 |

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 98 | $CH_3$ | Br | $CH_3$ | H | $CH_3$ | 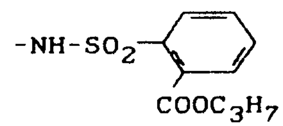 | 147 |
| 99 | $CH_3$ | Br | $CH_3$ | H | $CH_3$ | 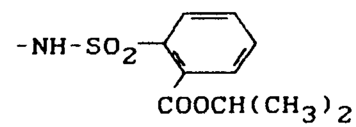 | 137 |
| 100 | $CH_3$ | Br | $CH_3$ | H | $CH_3$ | 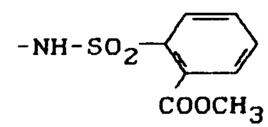 | 184 |
| 101 | $CH_3$ | Br | $CH_3$ | H | $CH_3$ | 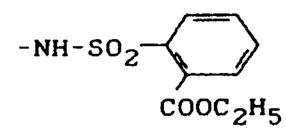 | 178 |
| 102 | $CH_3$ | Br | $CH_3$ | H | $CH_3$ | 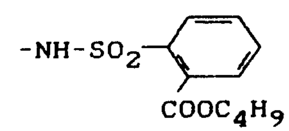 | 117 |
| 103 | $CH_3$ | Br | $CH_3$ | H | $CH_3$ | 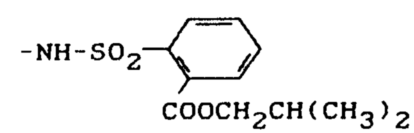 | 145 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 104 | $CH_3$ | Br | $CH_3$ | H | $CH_3$ | -N=CH- 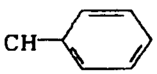 | 245 |
| 105 | $CH_3$ | Br | $CH_3$ | H | $CH_3$ | -N=CH- 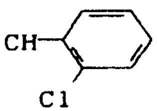 | 258 |
| 106 | $CH_3$ | Br | $CH_3$ | H | $CH_3$ | -N=CH- 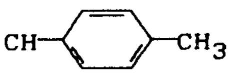 | 244 |
| 107 | $CH_3$ | Br | $CH_3$ | H | $CH_3$ | -N=CH- 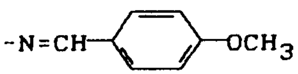 | 257 |
| 108 | $CH_3$ | Br | $CH_3$ | H | $OCH_3$ | -N=CH- 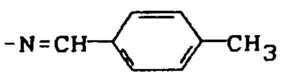 | 260 |
| 109 | $CH_3$ | Br | $CH_3$ | H | $OCH_3$ | -N=CH- 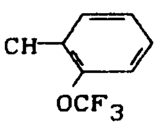 | 208 |
| 110 | $CH_3$ | Br | $CH_3$ | H | $OCH_3$ | -N=CH- 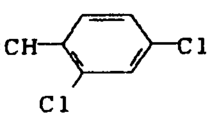 | 280 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 111 | $CH_3$ | Br | $CH_3$ | H | $OCH_3$ | 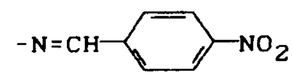 | 282 |
| 112 | $CH_3$ | Br | $CH_3$ | H | $CH_3$ | 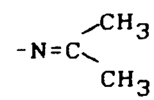 | 237 |
| 113 | $CH_3$ | Br | $CH_3$ | H | $CH_3$ | 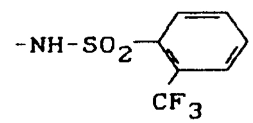 | 199 |
| 114 | $CH_3$ | Br | $CH_3$ | H | $CH_3$ | 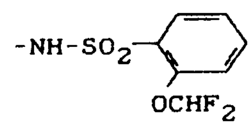 | 198 |
| 115 | $CH_3$ | Br | $CH_3$ | H | $CH_3$ | $-NH-SO_2-C_2H_5$ | 160 |
| 116 | $CH_3$ | Br | $CH_3$ | H | $CH_3$ | 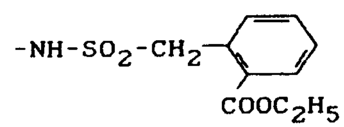 | 209 |
| 117 | $CH_3$ | Br | $CH_3$ | H | $CH_3$ | 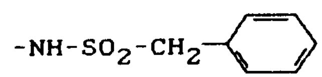 | 203 |

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 118 | $CH_3$ | Br | $CH_3$ | H | $CH_3$ | $-NH-SO_2-$ 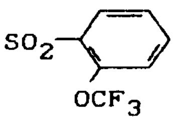 | 137 |
| 119 | $CH_3$ | Br | $CH_3$ | H | $CH_3$ | $-NH-SO_2-$ 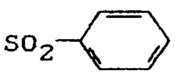 | 214 |
| 120 | $CH_3$ | Br | $CH_3$ | H | $CH_3$ | $-NH-SO_2-$ 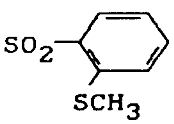 | 196 |
| 121 | $CH_3$ | Br | $CH_3$ | H | $CH_3$ | $-NH-SO_2-CH_2-$ 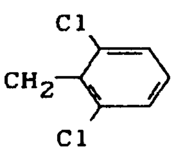 | 226 |
| 122 | $CH_3$ | $COOC_2H_5$ | $OCH_3$ | H | $OCH_3$ | $-NH-SO_2-CH_2-$ 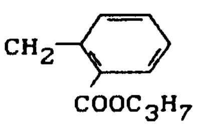 | 163 |
| 123 | $CH_3$ | $COOC_2H_5$ | $OCH_3$ | H | $OCH_3$ | $-NH-SO_2-CH_2-$ 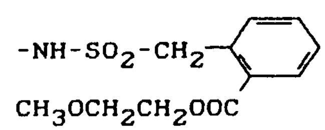 | 160 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 124 | $CH_3$ | $COOC_2H_5$ | $CH_3$ | H | $OCH_3$ | $-NH-SO_2-CH_2-$ 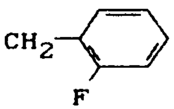 | 152 |
| 125 | $CH_3$ | $COOC_2H_5$ | $CH_3$ | H | $OCH_3$ | $-NH-SO_2-CH_2CH_2-$ 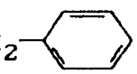 | 208 |
| 126 | $CH_3$ | $COOC_2H_5$ | $CH_3$ | H | $OCH_3$ | $-NH-SO_2-CH_2-$ 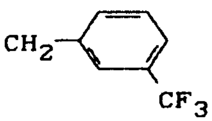 | 177 |
| 127 | $CH_3$ | $COOC_2H_5$ | $CH_3$ | H | $OCH_3$ | $-NH-SO_2-CH_2-$ 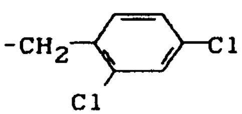 | 107 |
| 128 | $CH_3$ | $COOC_2H_5$ | $CH_3$ | H | $OCH_3$ | $-NH-SO_2-CH_2-$ 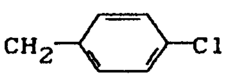 | 172 |
| 129 | $CH_3$ | $COOC_2H_5$ | $OCH_3$ | H | $OCH_3$ | $-NH-SO_2-CH_2-$ 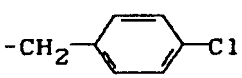 | 120 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 130 | $CH_3$ | $COOC_2H_5$ | $OCH_3$ | H | $OCH_3$ | $-NH-SO_2-CH_2-$ 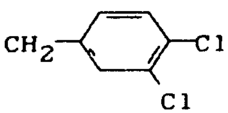 | 110 |
| 131 | $CH_3$ | $COOC_2H_5$ | $OCH_3$ | H | $OCH_3$ | $-NH-SO_2-CH_2-$ 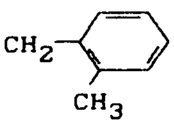 | 184 |
| 132 | $CH_3$ | $COOC_2H_5$ | $CH_3$ | H | $OCH_3$ | $-NH-SO_2-CH_2-$ 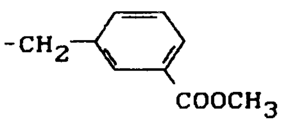 | 215 |
| 133 | $CH_3$ | Br | $OCH_3$ | H | $OCH_3$ | $-NH-CO-NH-C_3H_7$ | 210 |
| 134 | $CH_3$ | Br | $CH_3$ | H | $CH_3$ | $-NH-CO-NH-C_3H_7$ | 222 |
| 135 | $CH_3$ | Br | $CH_3$ | H | $CH_3$ | $-NH-CO-NH-$ 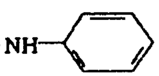 | 209 |
| 136 | $CH_3$ | Br | $CH_3$ | H | $OCH_3$ | $-NH-CO-NH-C_4H_9$ | 224 |
| 137 | $CH_3$ | Br | $CH_3$ | H | $OCH_3$ | $-NH-CO-NH-$ 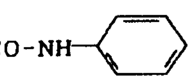 | 231 |

Tabelle 1 - Fortsetzung

| Bsp.-Nr. | $R^1$ | $R^2$ | $R^3$ | $R^4$ | $R^5$ | $R^6$ | Schmelz-punkt (°C) |
|---|---|---|---|---|---|---|---|
| 138 | $CH_3$ | Br | $OCH_3$ | H | $OCH_3$ | $-NH-CO-NH-C_4H_9$ | 196 |
| 139 | $CH_3$ | Br | $OCH_3$ | H | $OCH_3$ | $-NH-CO-NH-CH_3$ | 207 |
| 140 | $CH_3$ | Br | $OCH_3$ | H | $OCH_3$ | -NH-CO-NH 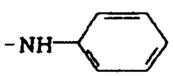 | 210 |
| 141 | $CH_3$ | Br | $CH_3$ | H | $OCH_3$ | $-NH-CO-NH-C_3H_7$ | 217 |
| 142 | $CH_3$ | Br | $CH_3$ | H | $OCH_3$ | $-NH-CO-NH-CH_3$ | 222 |
| 143 | $CH_3$ | $COOC_2H_5$ | $OCH_3$ | H | $OCH_3$ | $-NH-SO_2$ 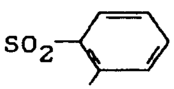 $COOC_3H_7$ | 205 |
| 144 | $CH_3$ | $COOC_2H_5$ | $OCH_3$ | H | $OCH_3$ | $-NH-SO_2$ 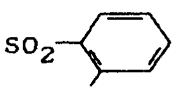 $COOCH(CH_3)_2$ | 229 |

Die Verbindungen der Beispiele Nr. 145-149 fielen bei der Herstellung gemäß Verfahren (b) als Pyridin-Salze an:

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 145 | $CH_3$ | $COOC_2H_5$ | $CH_3$ | H | $CH_3$ | $-NH-SO_2-$ 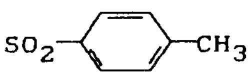 | 213 |
| 146 | $CH_3$ | $COOC_2H_5$ | $CH_3$ | H | $CH_3$ | $-NH-SO_2-CH_2-$ 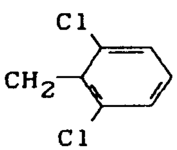 | 147 |
| 147 | $CH_3$ | Br | $CH_3$ | H | $OCH_3$ | $-NH-SO_2-$ 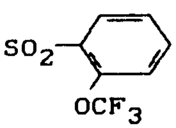 | 102 |
| 148 | $CH_3$ | Br | $CH_3$ | H | $OCH_3$ | $-NH-SO_2-$ 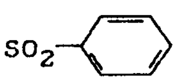 | 207 |
| 149 | $CH_3$ | Br | $CH_3$ | H | $OCH_3$ | $-NH-SO_2(CH_2)_4Cl$ | 182 |

<u>Tabelle 1</u> - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 150 | $CH_3$ | Br | $OCH_3$ | H | $OCH_3$ | $-NH-SO_2-CH_2-$ 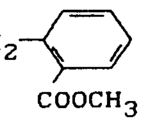 | 155 |
| 151 | $CH_3$ | $COOCH_3$ | $OCH_3$ | H | $OCH_3$ | $-NH-SO_2-CH_2-$ 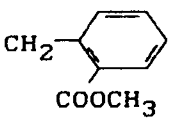 | 175 |
| 152 | $CH_3$ | Br | $CH_3$ | H | $OCH_3$ | $-NH-SO_2-CH_2-$ 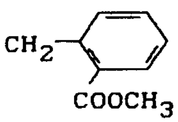 | 167 |
| 153 | $CH_3$ | Br | $CH_3$ | H | $OCH_3$ | $-NH-SO_2-$ 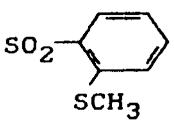 | 202 |
| 154 | $CH_3$ | Br | $CH_3$ | H | $OCH_3$ | $-NH-SO_2-N(CH_3)_2$ | 180 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 155 | $CH_3$ | $COOC_2H_5$ | $CH_3$ | H | $OCH_3$ | $-NH-SO_2-CH_2-$ 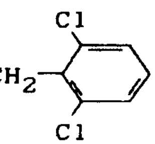 | 140 |
| 156 | $CH_3$ | Br | $CH_3$ | H | $CH_3$ | $-NH-SO_2-CH_3$ | 214 |
| 157 | $CH_3$ | $COOC_2H_5$ | $OCH_3$ | H | $OCH_3$ | $-NH-SO_2-CH_2-$ 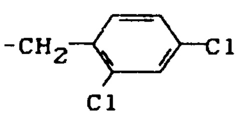 | 109 |
| 158 | $CH_3$ | Br | $OCH_3$ | H | $OCH_3$ | $-NH-SO_2-CH_2-$ 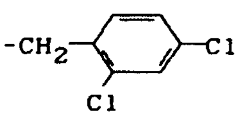 | 112 |
| 159 | $CH_3$ | Br | $CH_3$ | H | $OCH_3$ | $-NH-SO_2-CH_2-$ 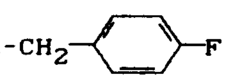 | 120 |

Tabelle 1 - Fortsetzung

| Bsp.-Nr. | $R^1$ | $R^2$ | $R^3$ | $R^4$ | $R^5$ | $R^6$ | Schmelzpunkt (°C) |
|---|---|---|---|---|---|---|---|
| 160 | $CH_3$ | Br | $CH_3$ | H | $OCH_3$ | $-NH-SO_2-CH_2-$ 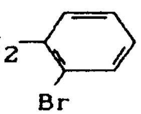 | 107 |
| 161 | $CH_3$ | $COOC_2H_5$ | $CH_3$ | H | $CH_3$ | $-NH-SO_2-CH_2-$ 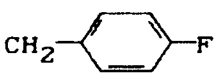 | 131 |
| 162 | $CH_3$ | $COOC_2H_5$ | $CH_3$ | H | $CH_3$ | $-NH-SO_2-CH_2-$ 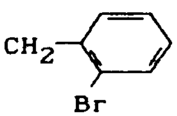 | 136 |

Ausgangsstoffe der Formel (II)

Beispiel (II-1)

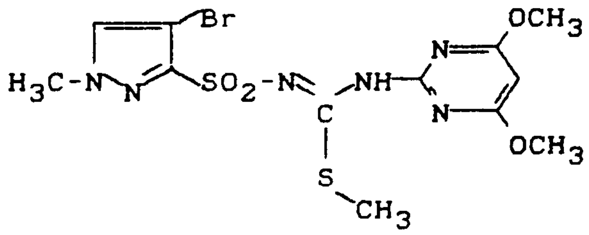

22,8 g (0,1 Mol) N-(4,6-Dimethoxy-pyrimidin-2-yl)-S-methyl-isothioharnstoff werden in 200 ml Methylenchlorid gelöst und auf -5 ° C abgekühlt. Dann werden unter Rühren 26 g (0,1 Mol) 4-Brom-1-methyl-pyrazol-3-sulfonsäurechlorid und 11,2 g 1,4-Diazabicyclo-[2,2,2]-octan in 50 ml Methylenchlorid dazu gegeben. Das Reaktionsgemisch wird eine Stunde bei -5 ° C und dann 20 Stunden bei +20 ° C gerührt. Nach Einengen im Wasserstrahlvakuum wird der Rückstand mit Wasser verrührt und das kristallin anfallende Produkt durch Filtrieren isoliert.

Man erhält 32,5 g (78% der Theorie) N-(4,6-Dimethoxy-pyrimidin-2-yl)-N'-(4-brom-1-methyl-pyrazol-3-yl-sulfonyl)-S-methyl-isothioharnstoff vom Schmelzpunkt 199 ° C.

Analog Beispiel (II-1) können beispielsweise auch die in der nachstehenden Tabelle 2 aufgeführten Verbindungen der Formel (II) erhalten werden.

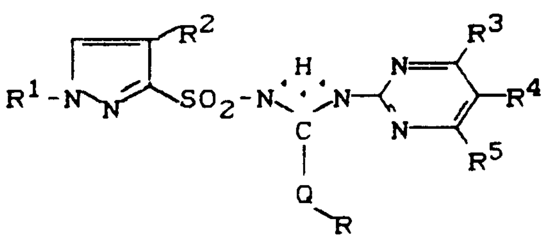

(II)

Tabelle 2

| Beispiele für die Verbindungen der Formel (II) | | | | | | | | |
|---|---|---|---|---|---|---|---|---|
| Bsp.-Nr., | Q | R | $R^1$ | $R^2$ | $R^3$ | $R^4$ | $R^5$ | Schmelzpunkt (° C) |
| II-2 | S | $CH_3$ | $CH_3$ | $COOC_2H_5$ | $OCH_3$ | H | $OCH_3$ | 172 |
| II-3 | S | $CH_3$ | $CH_3$ | Br | $CH_3$ | H | $OCH_3$ | 212 |
| II-4 | S | $CH_3$ | $CH_3$ | $COOC_2H_5$ | $CH_3$ | H | $OCH_3$ | 156 |
| II-5 | S | $CH_3$ | $CH_3$ | $COOC_2H_5$ | $CH_3$ | H | $CH_3$ | 205 |
| II-6 | S | $CH_3$ | $CH_3$ | Br | $CH_3$ | H | $CH_3$ | 197 |

Anwendungsbeispiele:

In den folgenden Anwendungsbeispielen wird die nachstehend aufgeführte Verbindung als Vergleichssubstanz herangezogen:

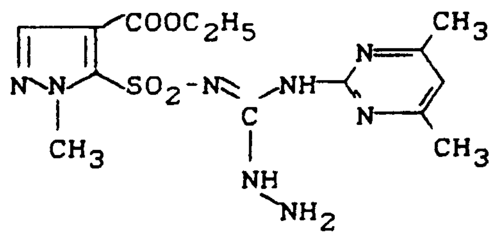

(A)

N'-(4,6-Dimethyl-pyrimidin-2-yl)-N''-amino-N'''-(1-methyl-4-ethoxycarbonyl-pyrazol-5-yl-sulfonyl)-guanidin (bekannt aus EP-A 343 462).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit gegenüber dem Stand der Technik zeigen in diesem Test z.B. die Verbindungen gemäß folgender Herstellungsbeispiele: 1, 6 und 15.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit gegenüber dem Stand der Technik zeigen in diesem Test z.B. die Verbindungen gemäß folgender Herstellungsbeispiele: 1, 7, 13, 16, 17, 20, 33 und 49.

## Patentansprüche

1. Substituierte Pyrazolylsulfonylguanidinopyrimidine der allgemeinen Formel (I)

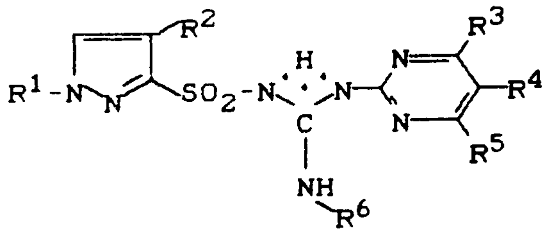

(I)

dadurch gekennzeichnet, daß

| | |
|---|---|
| $R^1$ | für jeweils gegebenenfalls substituiertes Alkyl, Alkenyl oder Alkinyl steht, |
| $R^2$ | für Wasserstoff, Halogen oder jeweils gegebenenfalls substituiertes Alkyl oder Alkoxycarbonyl steht, |
| $R^3$, $R^4$ und $R^5$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, und |
| $R^6$ | für Amino oder die Gruppierungen -NH-A-$R^7$ oder |

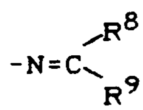

steht, worin

| | |
|---|---|
| A | für die Gruppierungen -CO-, -COO-, -CONH- oder -$SO_2$- steht, |
| $R^7$ | für jeweils gegebenenfalls substituiertes Alkyl, Aralkyl oder Aryl steht, |
| $R^8$ | für Wasserstoff oder für jeweils gegebenenfalls substituiertes Alkyl, Aryl oder Aralkyl steht, und |
| $R^9$ | für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^8$ für gegebenenfalls substituiertes Alkandiyl steht. |

2. Substituierte Pyrazolylsulfonylguanidinopyrimidine der allgemeinen Formel (I), dadurch gekennzeichnet, daß

| | |
|---|---|
| $R^1$ | für jeweils gegebenenfalls durch Halogen, Cyano, $C_1$-$C_4$-Alkoxy oder $C_1$-$C_4$-Alkoxycarbonyl substituiertes $C_1$-$C_4$-Alkyl, $C_2$-$C_4$-Alkenyl oder $C_2$-$C_4$-Alkinyl steht, |
| $R^2$ | für Wasserstoff, Halogen oder für jeweils gegebenenfalls durch Halogen oder $C_1$-$C_4$-Alkoxy substituiertes $C_1$-$C_4$-Alkyl oder $C_1$-$C_4$-Alkoxycarbonyl steht, |
| $R^3$, $R^4$ und $R^5$ | gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino stehen, und |
| $R^6$ | für Amino oder die Gruppierungen -NH-A-$R^7$ oder |

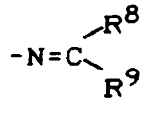

steht,
worin

A für die Gruppierungen -CO-, -COO-, -CONH oder $-SO_2-$ steht,

$R^7$ für gegebenenfalls durch Halogen substituiertes $C_1$-$C_{10}$-Alkyl oder für jeweils gegebenenfalls einfach bis dreifach substituiertes Benzyl, Phenylethyl, Phenyl oder Naphthyl steht, wobei die Substituenten vorzugsweise ausgewählt sind aus der Reihe Fluor, Chlor, Brom, Cyano, Nitro, Carboxy, Carbamoyl, jeweils gegebenenfalls durch Fluor, und/oder Chlor einfach bis dreifach substituiertes $C_1$-$C_4$-Alkyl, $C_1$-$C_4$-Alkoxy oder $C_1$-$C_4$-Alkylthio, jeweils gegebenenfalls durch Fluor, Chlor, Methoxy oder Ethoxy substituiertes $C_1$-$C_4$-Alkoxycarbonyl oder $C_1$-$C_4$-Alkylaminocarbonyl,

$R^8$ für Wasserstoff, für gegebenenfalls durch Fluor, Chlor und/oder Brom substituiertes $C_1$-$C_6$-Alkyl oder für jeweils gegebenenfalls durch Fluor, Chlor, Brom, $C_1$-$C_4$-Alkyl und/oder $C_1$-$C_4$-Alkoxy substituiertes Phenyl oder Benzyl steht, und

$R^9$ für Wasserstoff, für jeweils gegebenenfalls durch Fluor, Chlor und/oder Brom substituiertes $C_1$-$C_{20}$-Alkyl, $C_2$-$C_{10}$-Alkenyl, $C_2$-$C_{10}$-Alkinyl oder $C_4$-$C_{10}$-Alkadienyl, für $C_1$-$C_4$-Alkoxy-$C_1$-$C_4$-alkyl, für $C_1$-$C_4$-Alkylthio-$C_1$-$C_4$-alkyl, für gegebenenfalls durch Fluor, Chlor, Brom, Carboxy, Cyano, Nitro, Amino, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Di-($C_1$-$C_4$-alkyl)-amino, $C_1$-$C_4$-Alkoxycarbonyl, $C_1$-$C_4$-Alkoxy-$C_1$-$C_4$-alkoxycarbonyl und/oder Phenoxy (welches gegebenenfalls durch Fluor, Chlor und/oder Trifluormethyl substituiert ist) substituiertes Phenyl, für Naphthyl, für jeweils gegebenenfalls durch Cyano, Nitro, Fluor, Chlor, Brom, $C_1$-$C_4$-Alkyl und/oder $C_1$-$C_4$-Alkoxy substituiertes Pyridyl, Pyrrolyl, Furyl, Thiazolyl oder Thienyl, für Dithienyl, für jeweils gegebenenfalls durch Fluor, Chlor, Brom, $C_1$-$C_4$-Alkyl und/oder $C_1$-$C_4$-Alkoxy substituiertes Phenyl-$C_1$-$C_2$-alkyl oder Phenylethenyl, für $C_1$-$C_6$-Alkoxy, $C_1$-$C_6$-Alkoxycarbonyl oder Di-($C_1$-$C_4$-alkyl)-amino steht, oder zusammen mit $R^8$ für gegebenenfalls durch Fluor, Chlor, Brom, $C_1$-$C_4$-Alkyl und/oder $C_1$-$C_4$-Alkoxycarbonyl substituiertes $C_2$-$C_6$-Alkandiyl steht.

**3.** Substituierte Pyrazolylsulfonylguanidinopyrimidine der allgemeinen Formel (I) gemäß Anspruch 1, dadurch gekennzeichnet, daß

$R^1$ für Methyl, Ethyl, Propyl, Fluormethyl, Difluormethyl, Trifluormethyl, Chlormethyl, Dichlormethyl, Trichlormethyl, Fluorethyl, Difluorethyl, Trifluorethyl, Tetrafluorethyl, Pentafluorethyl, Chlorethyl, Dichlorethyl, Trichlorethyl, Chlorfluorethyl, Chlordifluorethyl, Chlortrifluorethyl, Cyanomethyl, Cyanoethyl, Methoxymethyl, Ethoxymethyl, Methoxyethyl, Ethoxyethyl, Methoxycarbonylmethyl, Ethoxycarbonylmethyl, Methoxycarbonylethyl, Ethoxycarbonylethyl, Allyl, Chlorallyl, Dichlorallyl, Trichlorallyl oder Propargyl steht,

$R^2$ für Wasserstoff, Chlor, Brom, Methyl, Methoxycarbonyl oder Ethoxycarbonyl steht,

$R^3$ für Wasserstoff, Fluor, Chlor, Brom, Methyl, Ethyl, Trifluormethyl, Chlormethyl, Methoxymethyl, Methoxy, Ethoxy, Difluormethoxy, Trifluorethoxy, Methoxyethoxy, Methylthio, Methylamino, Ethylamino oder Dimethylamino steht,

$R^4$ für Wasserstoff, Fluor, Chlor, Brom, Methyl, Trifluormethyl oder Methoxy steht,

$R^5$ für Wasserstoff, Fluor, Chlor, Brom, Methyl, Ethyl, Trifluormethyl, Methoxy, Ethoxy oder Difluormethoxy steht, und

$R^6$ für Amino oder die Gruppierungen
$-NH-A-R^7$ oder

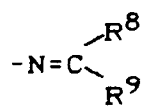

steht, worin

A für die Gruppierungen -CO-, -COO-, -CONH- oder $-SO_2-$ steht,

$R^7$ für gegebenenfalls durch Fluor oder Chlor substituiertes $C_1$-$C_6$-Alkyl oder für jeweils gegebenenfalls einfach oder zweifach substituiertes Benzyl oder Phenyl steht, wobei die Substituenten vorzugsweise ausgewählt sind aus der Reihe Fluor, Chlor, Brom, Carboxy, Methyl, Trifluormethyl, Methoxy, Difluormethoxy, Trifluormethoxy, Methoxycarbonyl, Ethoxycarbonyl,

$R^8$ für Wasserstoff, $C_1$-$C_4$-Alkyl oder Phenyl steht,

$R^9$ für $C_1$-$C_{10}$-Alkyl, $C_2$-$C_{10}$-Alkenyl oder $C_4$-$C_{10}$-Alkadienyl, für gegebenenfalls durch Fluor, Chlor, Brom, Carboxy, Cyano, Nitro, Methyl, Ethyl, Trifluormethyl, Methoxy, Ethoxy, Difluormethoxy, Trifluormethoxy, Methylthio, Ethylthio, Difluormethylthio, Trifluormethylthio, $C_1$-$C_4$-Alkoxycarbonyl, Dimethylamino, $C_1$-$C_2$-Alkoxy-$C_1$-$C_2$-alkoxycarbonyl und/oder Phenoxy (welches gegebenenfalls durch Fluor, Chlor und/oder Trifluormethyl substituiert ist) substituiertes Phenyl, für jeweils gegebenenfalls durch Cyano, Nitro, Fluor, Chlor, Brom, Methyl, Ethyl, Methoxy oder Ethoxy substituiertes Pyridyl, Furyl, Thiazolyl oder Thienyl, für Dithienyl, für Benzyl oder Phenylethenyl, für $C_1$-$C_4$-Alkoxy, $C_1$-$C_4$-Alkoxycarbonyl oder Dimethylamino, oder zusammen mit $R^8$ für Butan-1,4-diyl (Tetramethylen) oder Pentan-1,5-diyl (Pentamethylen) steht.

4. Verfahren zur Herstellung von substituierten Pyrazolylsulfonylguanidinopyrimidinen der allgemeinen Formel (I) gemäß Anspruch 1

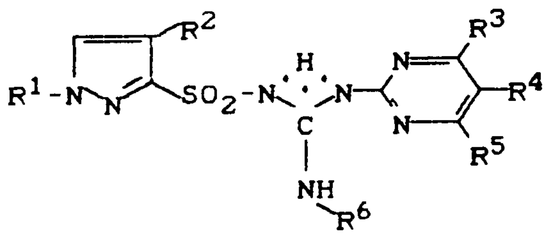

(I)

bei welchen

$R^1$ für jeweils gegebenenfalls substituiertes Alkyl, Alkenyl oder Alkinyl steht,

$R^2$ für Wasserstoff, Halogen oder jeweils gegebenenfalls substituiertes Alkyl oder Alkoxycarbonyl steht,

$R^3$, $R^4$ und $R^5$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen, und

$R^6$ für Amino oder die Gruppierungen
-NH-A-$R^7$ oder

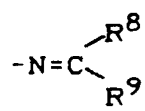

steht, worin

A für die Gruppierungen -CO-, -COO-, -CONH- oder -$SO_2$- steht,

$R^7$ für jeweils gegebenenfalls substituiertes Alkyl, Aralkyl oder Aryl steht,

$R^8$ für Wasserstoff oder für jeweils gegebenenfalls substituiertes Alkyl, Aryl oder Aralkyl steht, und

$R^9$ für Wasserstoff oder für jeweils gegebenenfalls substituiertes Alkyl, Alkenyl, Alkadienyl, Alkinyl, (Hetero)Aryl, Aralkyl, Aralkenyl, Alkoxy, Alkoxycarbonyl oder Dialkylamino steht, oder zusammen mit $R^8$ für gegebenenfalls substituiertes Alkandiyl steht,

dadurch gekennzeichnet, daß man

(a) Pyrazolylsulfonyl-iso(thio)ureidopyrimidine der allgemeinen Formel (II)

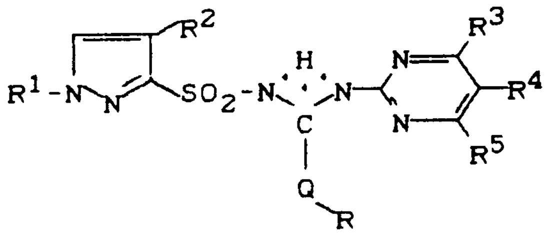

(II)

in welcher

$R^1$, $R^2$, $R^3$, $R^4$ und $R^5$ die oben angegebenen Bedeutungen haben,

Q für Sauerstoff oder Schwefel steht und

R für Alkyl steht,

mit Aminoverbindungen der allgemeinen Formel (III)

$H_2N$-$R^6$ (III)

in welcher

$R^6$ die oben angegebene Bedeutung hat,

gegebenenfalls in Gegenwart eines Verdünnungsmittels umsetzt, oder daß man

(b) für den Fall, daß in der Formel (I) $R^6$ für die Gruppierung -NH-A-$R^7$ steht und $R^1$, $R^2$, $R^3$, $R^4$, $R^5$, A und $R^7$ die oben angegebenen Bedeutungen haben,

Pyrazolylsulfonyl-aminoguanidinopyrimidine der allgemeinen Formel (Ia)

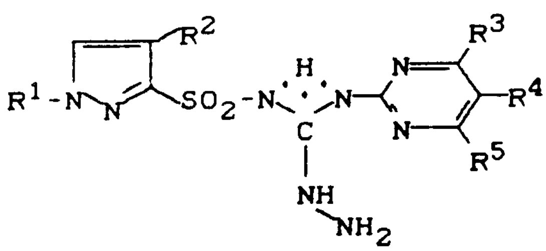

(Ia)

in welcher

$R^1$, $R^2$, $R^3$, $R^4$ und $R^5$ die oben angegebenen Bedeutungen haben,

mit Halogenverbindungen der allgemeinen Formel (IV)

X-A-$R^7$ (IV)

in welcher

A und $R^7$ die oben angegebenen Bedeutungen haben und

X für Halogen steht,

gegebenenfalls in Gegenwart eines Verdünnungsmittels und gegebenenfalls in Gegenwart eines Säureakzeptors umsetzt, oder daß man

(c) für den Fall, daß in der Formel (I) $R^6$ für die Gruppierung

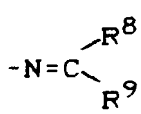

steht und $R^1$, $R^2$, $R^3$, $R^4$, $R^5$,

$R^8$ und $R^9$ die oben angegebenen Bedeutungen haben,
Pyrazolylsulfonyl-aminoguanidinopyrimidine der allgemeinen Formel (Ia)

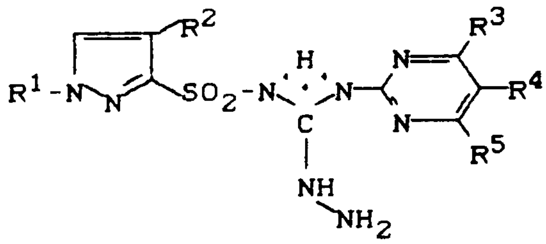

(Ia)

in welcher
$R^1$, $R^2$, $R^3$, $R^4$ und $R^5$ die oben angegebenen Bedeutungen haben,
mit Carbonylverbindungen der allgemeinen Formel (V)

$$R^8-\overset{\overset{\displaystyle O}{\|}}{C}-R^9 \qquad (V)$$

in welcher
$R^8$ und $R^9$ die oben angegebenen Bedeutungen haben,
oder mit N,N-Dialkyl-carbonsäureamid-acetalen bzw. -ketalen

gegebenenfalls in Gegenwart eines Kondensationshilfsmittels und gegebenenfalls in Gegenwart eines Verdünnungsmittels umsetzt.

**5.** Herbizide Mittel, gekennzeichnet durch einen Gehalt an mindestens einem substituierten Pyrazolylsulfonylguanidinopyrimidin der Formel (I) gemäß den Ansprüchen 1 bis 4.

**6.** Verfahren zur Bekämpfung von unerwünschten Pflanzen, dadurch gekennzeichnet, daß man substituierte Pyrazolylsulfonylguanidinopyrimidine der Formel (I) gemäß den Ansprüchen 1 bis 4 auf unerwünschte Pflanzen und/oder ihren Lebensraum einwirken läßt.

**7.** Verwendung von substituierten Pyrazolylsulfonylguanidinopyrimidinen der Formel (I) gemäß den Ansprüchen 1 bis 4 zur Bekämpfung von unerwünschten Pflanzen.

**8.** Verfahren zur Herstellung von herbiziden Mitteln, dadurch gekennzeichnet, daß man substituierte Pyrazolylsulfonylguanidinopyrimidine der Formel (I) gemäß den Ansprüchen 1 bis 4 mit Streckmitteln und/oder oberflächenaktiven Substanzen vermischt.

**9.** Pyrazolylsulfonyl-iso(thio)ureidopyrimidine der allgemeinen Formel (II)

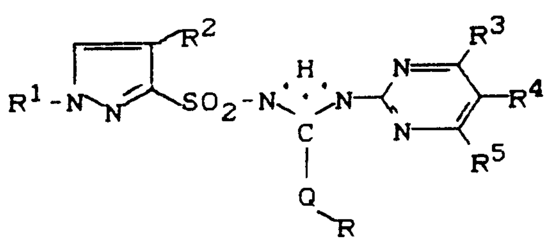

(II)

dadurch gekennzeichnet, daß
$R^1$ für jeweils gegebenenfalls substituiertes Alkyl, Alkenyl oder Alkinyl steht,
$R^2$ für Wasserstoff, Halogen oder jeweils gegebenenfalls substituiertes Alkyl oder

Alkoxycarbonyl steht,

$R^3$, $R^4$ und $R^5$ gleich oder verschieden sind und für Wasserstoff, Halogen, Alkyl, Halogenalkyl, Alkoxyalkyl, Alkoxy, Halogenalkoxy, Alkoxyalkoxy, Alkylthio, Alkylamino oder Dialkylamino stehen,

Q für Sauerstoff oder Schwefel steht und

R für Alkyl steht.

**10.** Pyrazolylsulfonyl-iso(thio)ureidopyrimidine der allgemeinen Formel (II)

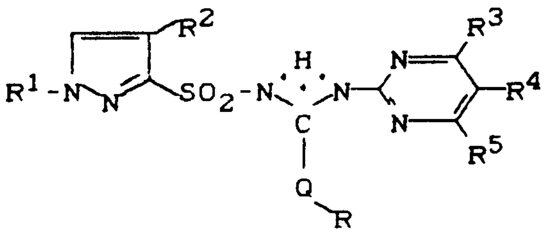

(II)

dadurch gekennzeichnet, daß

$R^1$ für jeweils gegebenenfalls durch Halogen, Cyano, $C_1$-$C_4$-Alkoxy oder $C_1$-$C_4$-Alkoxy-carbonyl substituiertes $C_1$-$C_4$-Alkyl, $C_2$-$C_4$-Alkenyl oder $C_2$-$C_4$-Alkinyl steht,

$R^2$ für Wasserstoff, Halogen oder für jeweils gegebenenfalls durch Halogen oder $C_1$-$C_4$-Alkoxy substituiertes $C_1$-$C_4$-Alkyl oder $C_1$-$C_4$-Alkoxy-carbonyl steht,

$R^3$, $R^4$ und $R^5$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_2$-Alkoxy-$C_1$-$C_2$-alkoxy, $C_1$-$C_4$-Alkylthio, $C_1$-$C_4$-Alkylamino oder Di-($C_1$-$C_2$-alkyl)-amino stehen,

Q für Sauerstoff oder Schwefel steht und

R für Methyl steht.

Europäisches Patentamt

# EUROPÄISCHER RECHERCHENBERICHT

Nummer der Anmeldung

EP 92 11 4288

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,D | EP-A-0 302 378 (BAYER AG)<br>* das ganze Dokument * | 1-8 | C07D403/12<br>A01N47/44 |
| X,D | EP-A-0 343 462 (BAYER AG)<br>* das ganze Dokument * | 1-8 | |
| X,D | EP-A-0 414 067 (BAYER AG)<br>* das ganze Dokument * | 1-8 | |
| A | EP-A-0 087 780 (NISSAN CHEMICAL INDUSTRIES LTD.)<br>* Ansprüche; Tabelle 3 * | 1-8 | |

RECHERCHIERTE SACHGEBIETE (Int. Cl.5)

C07D

Der vorliegende Recherchenbericht wurde für alle Patentansprüche erstellt

| Recherchenort | Abschlußdatum der Recherche | Prüfer |
|---|---|---|
| DEN HAAG | 27 OKTOBER 1992 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument